# EUROPÄISCHE PATENTSCHRIFT

(11) **EP 4 216 848 B1**
(45) Veröffentlichungstag und Bekanntmachung des Hinweises auf die Patenterteilung: **06.11.2024**
(21) Anmeldenummer: 21786806.6
(22) Anmeldetag: 28.09.2021
(51) Int. Cl.: A61B 17/70, A61B 17/86

(54) **MODULARE UND TEMPORÄR FIXIERBARE OSTEOSYNTHESEVORRICHTUNG FÜR WIRBEL**
MODULAR AND TEMPORARILY FIXABLE OSTEOSYNTHESIS DEVICE FOR VERTEBRAE
DISPOSITIF D'OSTÉOSYNTHÈSE MODULAIRE ET À FIXATION TEMPORAIRE POUR VERTÈBRES

(30) Priorität: 28.09.2020 DE 102020005928
(43) Veröffentlichungstag der Anmeldung: 02.08.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/076697
(87) Internationale Veröffentlichungsnummer: WO 2022/064070

(56) Entgegenhaltungen:
- EP-A1- 2 022 423
- EP-A1- 3 287 088
- EP-A1- 3 437 576
- US-A1- 2009 149 887

## Beschreibung

### Stand der Technik

Im Stand der Technik sind verschiedene Osteosynthesevorrichtungen zur Versorgung der Wirbelsäule, wie beispielsweise Pedikelschrauben, bekannt. Solche Osteosynthesevorrichtungen werden dafür verwendet Wirbelsäulenfehlstellungen zu korrigieren oder Frakturen zu stabilisieren, indem die Osteosynthesevorrichtungen in den Wirbelknochen eingebracht und befestigt werden und dann über Längsstäbe, oder sogenannten Verbindungsstäbe, miteinander verbunden werden, um die Wirbel in einer gewünschten Stellung zu fixieren. Dabei werden die Längsstäbe mit Hilfe von Madenschrauben an den Osteosynthesevorrichtungen montiert und rutschfest fixiert. Als Osteosynthesevorrichtungen kommen vorzugsweise Pedikelschrauben zum Einsatz, die einen Knochenanker besitzen, welcher polyaxial mit einem Gabelkopf verschwenkbar gelagert ist und bei fixierter Madenschraube winkelstabil ist. Als Knochenanker kommen vorzugsweise Knochenschrauben mit einem Kugelkopf zum Einsatz. Regulär werden Osteosynthesevorrichtungen mit Knochenanker und Gabelkopf so montiert, dass der Knochenanker von proximal kommend in den Gabelkopf und der Knochenankerschaft durch die distale Öffnung des Gabelkopfes geführt wird. Das funktioniert nur, wenn der äußere Durchmesser des Knochenankerschafts kleiner ist als der Kugelkopfdurchmesser des Knochenankers und der äußere Durchmesser des Knochenankerschafts kleiner ist als der Durchmesser der distalen Öffnung des Gabelkopfes. Problematisch ist die Montage, wenn der äußere Durchmesser des Knochenankerschafts größer ist als der Öffnungsdurchmessers der Gabelkopfes und/oder des Kugelkopfdurchmesser des Knochenankers.

Aus dem Stand der Technik ist beispielsweise aus der US20060200128A1 oder US2020121367A1 ein Aufbau einer Pedikelschraube bekannt, bei der der Gabelkopf von distal kommend mit einem Knochenanker montiert werden kann. Hierbei ist der Gabelkopf mehrteilig aufgebaut und besitzt eine Art Spannkäfig oder Spannzange am distalen GabelkopfBereich. Oft sind drei oder mehr Bauteile notwendig. Ein mehrteiliger Aufbau eines Knochenankers wirkt sich nachteilig bezüglich der mechanischen Stabilität und maximalen Belastbarkeit aus. Eine Überlastung kann zur Demontage und damit zum frühzeitigem Implantatversagen führen. Deshalb wäre es Aufbau mit so wenigen Komponenten wie möglich erstrebenswert, da es auch die Anzahl möglicher Fehler in der Herstellung reduziert. Die US20090149887A1 offenbart eine Vorrichtung zum Verbinden eines Knochenankers mit einer Stützstange, umfassend einen Verbindungskörper und eine Kappe, wobei der Verbindungskörper einen Kanal zur Aufnahme und Positionierung der Stützstange relativ zum Knochenanker hat, und die Kappe in Längsrichtung in eine teilweise installierte Position im Kanal beweglich ist und rastet in einen gewindelosen Eingriff mit dem Verbinderkörper ein, indem sie von der teilweise installierten Position in eine installierte Position gedreht wird, ohne sich axial relativ zum Verbinderkörper zu bewegen. In einigen Ausführungsformen passt eine Hülse über einen Sockelabschnitt des Verbinderkörpers in einer vorübergehenden Position, in der die Hülse das Einsetzen des Knochenankers in den Sockel ermöglicht. Die Hülse greift in den Verbinderkörper ein, so dass sie in der vorübergehenden Position an einer axialen und rotatorischen Bewegung relativ zum Verbinderkörper gehindert wird. Des Weiteren sind aus dem Stand der Technik Pedikelanker (DE102018102173B3) bekannt, die temporär verriegelbar sind und somit ein erweitertes Anwendungssprektrum bei der Versorgung von Wirbelsäuleninstabilitäten erlauben. Eine solche Anordnung ist technologisch und aus Sicht der Herstellung aufwendig und kostenintensiv. Erstrebenswert ist es deshalb, so wenig wie möglich Bauteile vorzusehen, eine temporäre Klemmbarkeit zu implementieren, die Montageschritte zu reduzieren und gleichzeitig die Herstellkosten zu optimieren.

### Darstellung der Erfindung

Die Erfindung betrifft eine Osteosynthesevorrichtung mit den Merkmalen des unabhängigen Anspruchs 1.

Vorteilhafte Weiterbildungen der Erfindung sind Gegenstand der abhängigen Ansprüche.

Die Erfindung betrifft eine Osteosynthesevorrichtung, insbesondere polyaxiale Pedikelschraube, mit einen Kopf aufweisenden Knochenanker und mit einem in einer Seitenansicht U-förmigen Gabelkopf, mit daran befestigten Verriegelungsring und eine am Gabelkopf befindlichen Aufnahmeöffnung für einen Verbindungsstab, insbesondere ein Korrekturstab, und ein im Gabelkopf geführten Verschlusselement. Als Knochenanker kommen vorzugsweise Knochenschrauben zum Einsatz, die mit einem Knochen verschraubbar sind. Es sind aber auch Haken, Klemmen, Nägel und andersartig gestaltete Knochenanker anwendbar. Die wesentlichen Merkmale des Knochenankers sind ein kugelähnlicher Kopf, ein Halsbereich und ein Bereich, welcher im oder am Knochen verankert oder befestigt werden kann. In dieser Patentanmeldung soll hauptsächlich auf den Gabelkopf eingegangen werden, und mit Knochenanker sollen alle erdenklichen mit einen Knochen verbindbaren Elemente verstanden werden.

Ein wesentliches Merkmal des Gabelkopfes ist, dass der Kugelkopfaufnahmebereich nach distal offene Schlitze aufweist und dadurch wenigstens ein auslenkbarer Schenkel ausgebildet ist. Die Schenkel bilden nach innen den Kugelsitz für die Knochenankerkopf aus. Werden die Schenkel nach außen flexiert geben sie den Kugelkopf des Knochenankers frei. Werden die Schenkel nach innen flexiert, generieren sie eine Kompressionskraft auf den Kugelkopf des Knochenankers und erzeugen eine Klemmung. Die Schenkel besitzen an der Außenseite einen Konus-Abschnitt über welche die Kompressionskraft eingeleitet werden kann. Des Weiteren besitzt die Außenkontur einen zylindrischen Bereich, der dazu dient, dass ein nicht-Kraft-beaufschlagter Formschluss zwischen Verriegelungsring und Kugelkopfaufnahmebereich des Gabelkopfs eingestellt werden kann. Kugelkopfaufnahmebereich und Verriegelungsring besitzen am Formschluss regelmäßig wiederkehrende Vertiefungen, so dass bei Verdrehung des Verriegelungsrings dieser Formschluss deaktiviert werden kann.

Des Weiteren besitzt die Osteosynthesevorrichtung einen Verriegelungsring, der zumindest teilweise um den Kugelkopfaufnahmebereich des Gabelkopfs herum angebracht ist, so dass der Verriegelungsring um eine Zentralachse gegenüber dem Gabelkopf drehbar ist. Es kann eine erste Drehstellung (R1) eingestellt werden, bei welcher der Kugelkopfaufnahmebereich mit seinen Schenkeln nach radial außen auslenkbar ist, so dass der Knochenanker entfernbar und/oder einsteckbar ist, und eine zweite Drehstellung (R2) eingestellt werden kann, bei welcher der Kugelkopfaufnahmebereich mit seinen Schenkeln in seiner Auslenkbarkeit blockiert ist, so dass der Knochenanker ortsfest aber schwenkbar gehalten wird. Durch Verdrehen des Verriegelungsrings in die R2 Drehstellung wird ein Formschluss zwischen der maximalen äußeren zylindrischen Kontur des Gabelkopfs und der kleinsten zylindrischen Innenkontur des Verriegelungsrings erzeugt. Dieser Formschluss verhindert jegliche Aufspreizung des Kugelkopfaufnahmebereichs und so ist der Knochenanker verliersicher aber beweglich im Gabelkopf gelagert. Das Verdrehen in eine andere Richtung (R1) öffnet diesen Formschluss wieder und der Kugelkopfaufnahmebereich ist verformbar, so dass der Knochenanker entfernbar ist.

Aufgrund dieser besonders positiven Eigenschaft ist der Gabelkopf dazu geeignet unterschiedliche Stabdurchmesser aufzunehmen. Befindet sich der Verriegelungsring in der R1-Drehstellung, sind durch diese vorteilhafte Anordnung der Komponenten, die Knochenanker durch Aufsetzen bzw. Aufpressen relativ einfach mit dem Gabelkopf montierbar. Durch ein Hilfsmittel, wie beispielsweise einem Löse-Instrument, ist der Knochenanker auch wieder entfernbar. Damit kann die erfindungsgemäße Osteosynthesevorrichtung modular vom Anwender konfiguriert und im Operationssaal zu einem späteren Zeitpunkt, als der der Fertigung, zusammengesetzt werden. Dadurch ist es beispielsweise möglich, dass zuerst der Knochenanker einzeln in den Knochen verankert oder geschraubt wird, und dann im Anschluss der Gabelkopf mit der Verriegelungsring am bereits implantierten Knochenanker befestigt wird. Dies hat den großen Vorteil, dass der Chirurg nach der Implantation des Knochenankers deutlich mehr im Platz und eine bessere Sicht im OP-Feld im Vergleich zu den sonst vollständig implantierten Pedikelschrauben hat. Nach der Montage wird der Verriegelungsring in die Drehstellung R2 gebracht und die polyaxiale Pedikelschraube kann wie gewohnt implantiert werden.

Vorteil ist, dass einerseits größere Knochenanker, d.h. Knochenanker mit einem größeren Außendurchmesser, als der distale Innendurchmesser des Gabelkopfes montiert werden können. Andererseits kann das Knochenanker-Portfolio minimalisiert werden, da der Anwender Gabelkopf und Knochenanker während der Operation mit einander kombinieren kann, anstatt auf ein vorgefertigtes übergroßes Portfolio zurückzugreifen. Ein solches Portfolio muss beim Anwender vorrätig sein und somit ist deutlich mehr Kapital gebunden, als es die erfindungsgemäße modulare Version erfordern würde.

Die Drehung des Verriegelungsrings wird üblicherweise mit einem Instrument durchgeführt, welches hier zur Vereinfachung der Darstellungen weggelassen wurde. Dieses Instrument hält beide Komponenten in einer H1-Stellung und erlaubt eine Rotation (z.B. R1 zu R2 oder umgekehrt) der beiden Komponenten zueinander. Optimalerweise wird die Osteosynthesevorrichtung vom Instrument erst wieder freigegeben, wenn die Drehstellung R2 eingestellt ist, damit die Osteosynthesevorrichtung für die Anwendung ordnungsgemäß montiert ist.

In der bevorzugten Ausgestaltungsform ist es auch möglich den Klemmeffekt über einen Vorsprung beziehungsweise eine proximale Kontaktstelle an der Außenwandung des Verriegelungsrings einzuleiten ohne dass der Verbindungsstab oder die Madenschraube vorhanden sind. Da es sich nicht um eine finale Klemmung mit eingelegtem Verbindungsstab handelt, nennt sich diese Art der Klemmung, temporäre Klemmung. Mit der temporären Klemmung ist es für den Anwender möglich, während der Operation eine polyaxiale Schraube in einer gewünschten Winkelstellung in eine monoaxiale Schraube zu konvertieren. Das heißt, es sind alle rotatorischen Freiheitsgrade einer polyaxialen Schraube temporär gesperrt. Die Schraube verhält sich monoaxial. Damit kann der Anwender nun den zu behandelten Wirbel translatorisch als auch rotatorisch solange manipulieren, bis er in der gewünschten Endstellung einen Verbindungsstabe einlegt und mit der Madenschraube fixiert. Mit einer polyaxialen Schraube sind solche Korrektionsmanöver nicht möglich, da eine von Patienten-seitig außen initiierte Korrektionsmanöver in einer freien Bewegung des polyaxial-Kugel-Gelenks resultiert und somit nicht an den Wirbel weitergeleitet wird. Dies funktioniert nur mit deaktivierten rotatorischen Freiheitsgraden im Kugelgelenk, also temporär geklemmt ist.

Unabhängig von der temporären Klemmung, muss nach der Implantation der Osteosynthesevorrichtung in den Knochen, ein Verbindungsstab eingelegt und mit Hilfe eines Verschlusselements die Osteosynthesevorrichtung final in allen Freiheitsgraden fixiert werden. Dies geschieht durch Festschrauben des Verschlusselements. Bei fest angezogenen Verschlusselement wird eine axiale Kompressionskraft vom Verschlusselement auf den Verbindungsstab übertragen, und dieser drückt auf die Stabauflagerstellen des Verriegelungsrings und erzeugt eine Relativbewegung des Verriegelungsrings weiter nach distal, so dass der Kugelkopfaufnahmebereich über eine paarige Konusverbindung vom Verriegelungsring derart zusammengedrückt wird, so dass der Knochenanker winkelstabil im Kugelsitz geklemmt wird. Optimalerweise werden zwei oder mehr Osteosynthesevorrichtungen mit Hilfe eines Verbindungsstabs miteinander verbunden.

Am proximalen Gabelkopfbereich ist eine umlaufene Nut mit einem hakenähnlichen Profil vorgesehen, die einen Hintergriff für ein Instrument bereitstellt. Stellvertretend sind andersartig gestaltete Nutprofile oder auch andere Haltemerkmale wie z.B. Öffnungen denkbar, die einen Hintergriff für ein Instrument bereitstellen.

Am proximalen Ende des Gabelkopfs können sich weitere und lösbare Abschnitte mit einem Gewindebereich befinden, die eine Reposition des Verbindungsstabs erlauben. Es ist auch denkbar, dass eine durch zwei längere Schenkel ausgebildeter hülsenähnlicher Zugang vorgesehen ist, wie es für den minimal-invasiven Zugang zum Einsatz kommt. Dabei können die lösbaren Schenkelverlängerungen optional am proximalen Ende miteinander verbunden sein. Mit lösbarer Verbindung sind beispielsweise Sollbruchstellen gemeint, die dazu geeignet sind die Verlängerungen nach final fixiertem Verbindungsstab zu entfernen.

Als Material eignen sich alle metallischen Legierungen, die als orthopädischer Implantatwerkstoff bekannt und akzeptiert sind. Dazu gehören beispielsweise Titan-, Cobalt-Chrom und Edelstahllegierungen. Sollte die herkömmliche Herstellung des Gabelkopfes und des Verriegelungsrings nicht oder nur unter höchsten technologischen Aufwand möglich sein, stellt die additive Fertigung das Mittel der Wahl dar. Somit kann der Gabelkopf und/oder der Verriegelungsring einstückig aufgebaut werden. Additive Fertigungen von metallischen Legierungen, oder auch 3D-Druck genannt, greifen auf das Laser- oder Elektronenstrahl-Schmelzverfahren zurück.

Der Langzeit-Erfolg eines 3d-gedruckten Implantats ist stark von seiner Nachbehandlung abhängig. Eine gezielte Wärmebehandlung (z.B. HIP - hot isostatic pressing) und Oberflächenbehandlung sind enorm wichtig. Hierzu ist einschlägige Literatur verfügbar, die die Zusammenhänge der Nachbehandlungen darlegen. Aufgrund der schlecht zugänglichen und feinmechanischen Merkmale ist ein Schleif- und Strahlverfahren nicht zielführend. Hier kann mit Hilfe des chemischen Ätzens, welches optional durch eine galvanische Spannung und/oder durch mechanische Stimulation unterstützt werden kann, eine entsprechende Reduktion der Oberflächenrauigkeit erzielt werden. Ziel ist es die Komponenten von unvollständig verschweißten Partikeln zu befreien, da hier Zugspannungen auftreten und Mikro-Kerben, die durch die unvollständig-verschweißten Partikel entstehen, eine Schwächung der Dauerfestigkeit bedeuten können.

Weitere Merkmale, Vorteile und Einzelheiten der Erfindung ergeben sich aus den beigefügten Patentansprüchen und der zeichnerischen Darstellung und nachfolgenden Beschreibung bevorzugter Ausführungsformen der erfindungsgemäßen Osteosyntheseschraube.

### Kurze Beschreibung der Zeichnungen zeigen

Fig. 1 eine Schrägansicht der erfindungsgemäßen Osteosynthesevorrichtungen,
Fig. 2a eine Explosionsdarstellung einer erfindungsgemäßen Osteosynthesevorrichtung bestehend aus einem Gabelkopf und einem Verriegelungsring,
Fig. 2b und 2c die Montage mit einem Knochenanker,
Fig. 3a eine Schrägansicht der final montierten erfindungsgemäßen Osteosynthesevorrichtung und das Einlegen eines Verbindungsstabs und einem Verschlusselement,
Fig. 3b die erfindungsgemäße Osteosynthesevorrichtung final winkelstabil fixiert mit dem Verschlusselement
Fig. 4a die Gabelkopfkomponenten in einer Schrägansicht, und
Fig. 4b in einer alternativen Schrägansicht
Fig. 5 den Gabelkopf in einer Seitenansicht mit einem dazugehörigen Schnitt
Fig. 6 den Verriegelungsring in einer Schrägansicht
Fig. 7 den Verriegelungsring in einer Draufsicht mit zwei Schnittansichten
Fig. 8a und 8b zeigen den Gabelkopf mit Verriegelungsring in einer Position- bzw. Drehstellung, in der ein Knochenanker entfernbar oder montierbar ist
Fig. 9a und 9b zeigen den Gabelkopf mit Verriegelungsring in einer Position- bzw. Drehstellung, in der ein Knochenanker im Kugelkopfaufnahmebereich durch einen gegenseitigen Formschluss gehalten wird.
Fig. 10 und Fig. 11 stellen den Gabelkopf und den Verriegelungsring in einer Seitenansicht mit verschiedenen Schnittdarstellungen dar
Fig. 12a und 12b zeigen die Demontage des Verriegelungsrings vom Gabelkopf
Fig. 13a bis c zeigen eine alternative Ausgestaltungsform von Gabelkopf und Verriegelungsring
Fig. 14 illustriert eine alternative Ausgestaltungsform
Fig. 15 demonstriert eine weitere alternative Ausgestaltungsform mit proximal geschlossenen Gabelschenkeln
Fig. 16a und b stellen die Verwendung eines Instruments zur temporären Fixierung dar.

### Beschreibung der bevorzugten Ausführungsformen

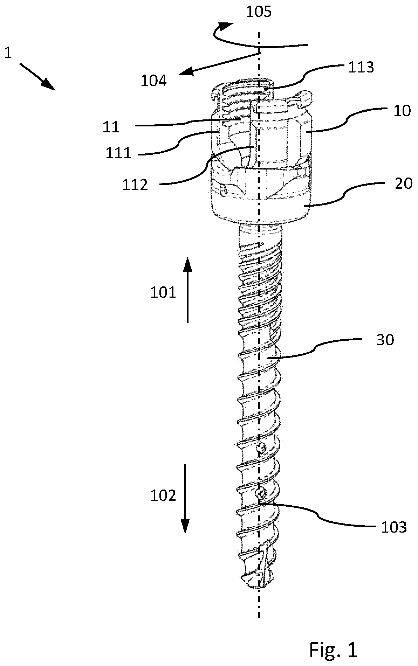

Beschrieben wird eine Osteosynthesevorrichtung (1), zur Behandlung der Wirbelsäule, wobei mehr als eine Osteosynthesevorrichtung (1) eingesetzt wird, um ein oder mehr Wirbel mit Hilfe von Verbindungsstäben (40) miteinander zu verbinden und somit die Wirbelsäule zu stabilisieren. Für die Osteosynthesevorrichtung (1), insbesondere für den Gabelkopf (10) sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert die radiale Ausbreitung (104) und die umfängliche Ausbreitung (105) ist durch einen konstanten Radius und einen variablen Umfangswinkel definiert (Fig. 1).

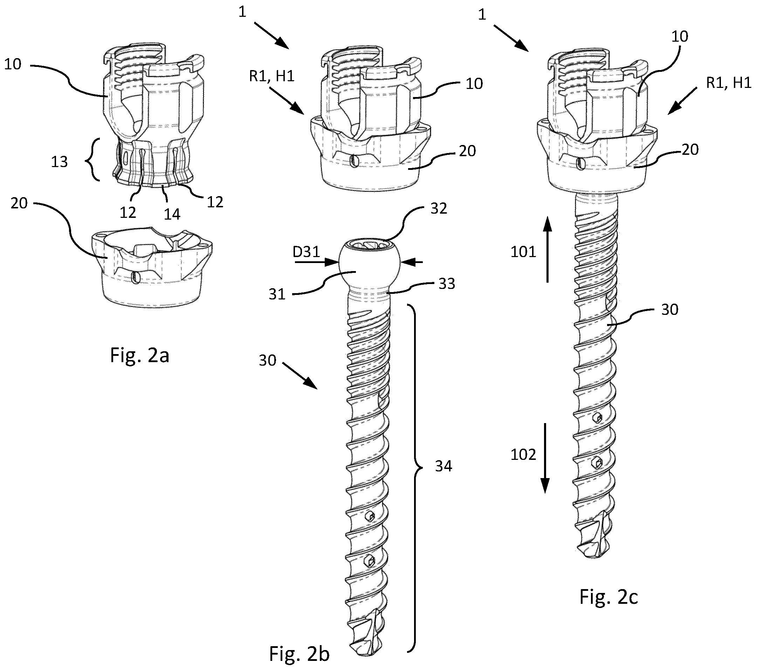

Die Osteosynthesevorrichtung (1) besitzt dabei einen in der Seitenansicht u-förmigen Gabelkopf (10), der in proximaler Richtung (101) zwei Gabelschenkel (111, 112) mit einem innenliegenden Gewinde (113) aufweist, und darin ein Verbindungsstab (40) aufgenommen werden kann, und in dem innenliegenden Gewinde (113) ein Verschlusselement bzw. Madenschraube (60) geführt ist, und der Gabelkopf (10) lösbar mit einem Knochenanker (30) verbunden ist (Fig. 1, Fig. 2b und 2c), und der Knochenanker (30) schwenkbar im Kugelkopfaufnahmebereich (13) des Gabelkopfs gelagert (10) ist, wobei der Gabelkopf (10) am Kugelkopfaufnahmebereich (13) nach distal offene Schlitze (12) aufweist und dadurch wenigstens zwei auslenkbarer Schenkel (14) ausgebildet ist (Fig. 2a).

Wobei der Knochenanker (30), einen Kopf (31), eine Werkzeugansatzstelle (32), einen Halsbereich (33) sowie ein optionales Knochengewinde (34) besitzt. Der Kopf (31) ist durch eine kugelähnliche Außenfläche charakterisiert, welche durch einen Durchmesser D31 beschreibbar ist (Fig. 2b).

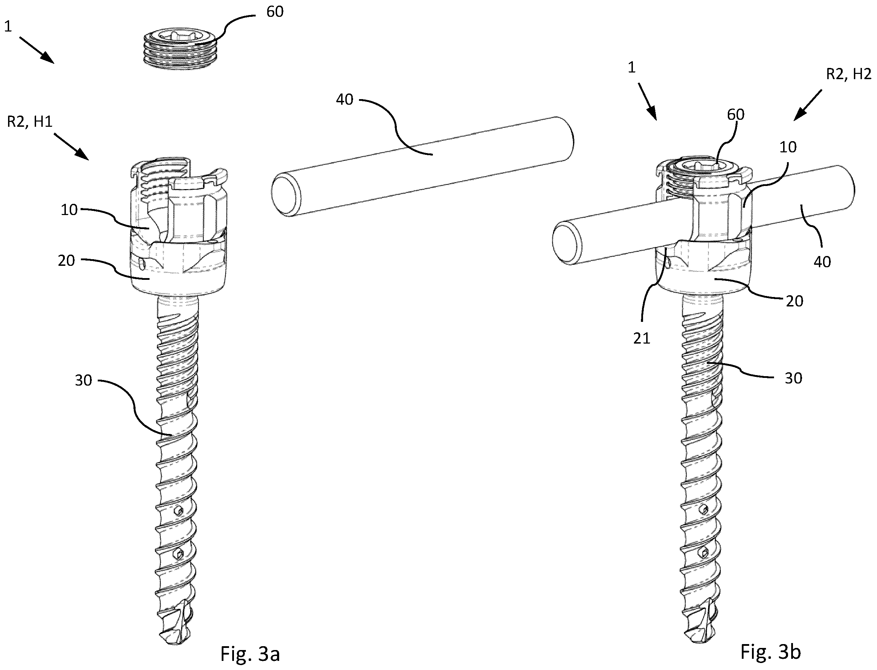

Des Weiteren besitzt die Osteosynthesevorrichtung (1) einen Verriegelungsring (20), der zumindest teilweise um den Kugelkopfaufnahmebereich (13) herum angebracht ist, so dass der Verriegelungsring (20) um eine Zentralachse (103) gegenüber dem Gabelkopf (10) drehbar ist (Fig. 2c und 3a). Es kann eine erste Drehstellung (R1) eingestellt werden, bei welcher der Kugelkopfaufnahmebereich (13) mit seinen Schenkeln (14) nach radial außen auslenkbar (57) ist, so dass der Knochenanker (30) entfernbar und/oder einsteckbar ist, und es eine zweite Drehstellung (R2) eingestellt werden kann, bei welcher der Kugelkopfaufnahmebereich (13) mit seinen Schenkeln (14) in seiner Auslenkbarkeit blockiert (58) ist, so dass der Knochenanker (30) ortsfest aber schwenkbar gehalten wird. Auf die genaue Funktion der Drehstellungen und Positionen des Verriegelungsrings wird in den nachfolgenden Abbildungen eingegangen.

Als Verschlusselement können beispielsweise Madenschrauben (60) verwendet werden (Fig. 3a und 3b). Typischerweise besitzen sie eine Werkzeugansatzstelle, ein Außengewinde und eine distale Kontaktfläche, welche im montierten Zustand in Kontakt zum Verbindungsstab (40) steht. Vorzugsweise besitzt das Außengewinde der Madenschraube einen Hinterschnitt, so dass beim Anziehen der Madenschrauben die beiden Gabelkopfschenkel (111 und 112) sich nicht nach radial außen verformen.

In Fig. 3b dargestellt ist der Zustand der Osteosynthesevorrichtung (1), wenn sie mit einem Verbindungsstab final fixiert ist. Bei fest angezogenen Verschlusselement (60) wird eine axiale Kompressionskraft vom Verschlusselement (60) auf den Verbindungsstab (40) übertragen, und dieser drückt auf die Stabauflagerstellen (21) des Verriegelungsrings (20) und erzeugt eine Relativbewegung des Verriegelungsrings (20) weiter nach distal, so dass der Kugelkopfaufnahmebereich (13) vom Verriegelungsring (20) derart zusammengedrückt wird, so dass der Knochenanker (30) winkelstabil im Kugelsitz (15) geklemmt wird.

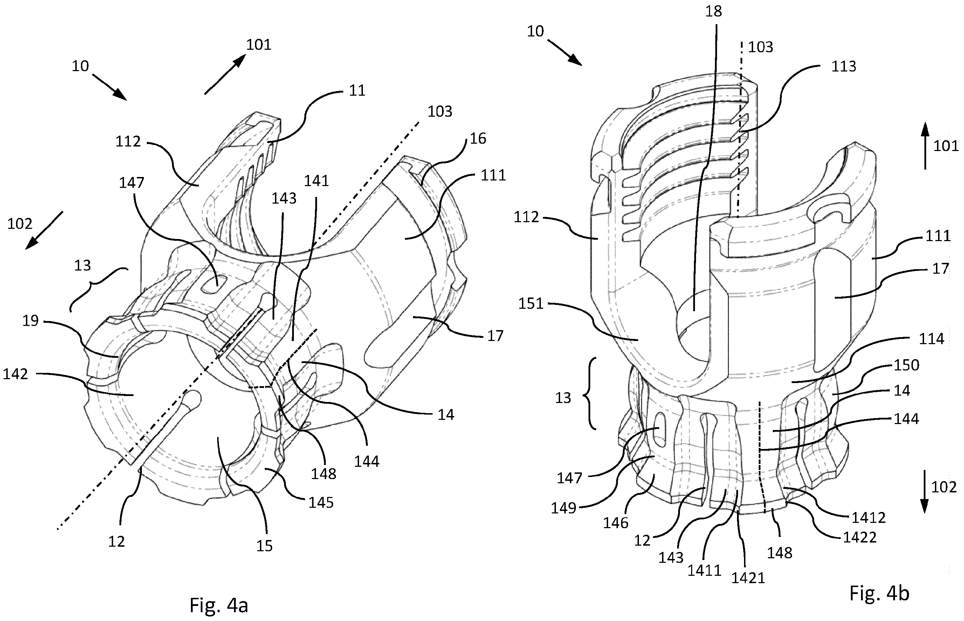

Fig. 4a und 4b zeigen den Aufbau der bevorzugten Ausführungsform des Gabelkopfes (10). Zu erkennen ist, dass am distalen Ende (102) des Gabelkopfes durch Schlitze getrennte Schenkel (14) gebildet sind. Die Schenkel (14) besitzen eine Außenkontur (141), welche den Kugelkopfaufnahmebereich (13) beschreiben. Des Weiteren besitzen die Schenkel (14) in Umfangsrichtung (105) wenigstens eine Vertiefung (143), die sich zumindest abschnittsweise hauptsächlich entlang der Zentralachse (103) erstreckt. Diese Vertiefungen (143) gehen in die Außenkontur (141) der Schenkel (14) über Rundungen und/oder Schrägen (1411, 1412, 1421, 1422) oder sonstigen Teilflächen ineinander über. Vorteilhaft ist, wenn die Vertiefungen (143) sich symmetrisch von einer gedachten Schenkelmittellinie (144) in Umfangsrichtung (105) erstrecken und durch Schlitze (12) geteilt und/oder unterbrochen sind. Dies führt dazu, dass die Schenkel (14) entlang der gedachten Schenkelmittellinie (144) dicker sind als die Vertiefungsbereiche (143), die zu den Spalt-angrenzenden Bereichen (12) verlaufen.

In den Abbildungen Fig. 4a und 4b ist ebenfalls dargestellt, dass am distalen Ende (102) des Gabelkopfes (10) die Schlitze (14) einen Abschnitt entlang der Zentralachse (103) bilden, welcher eine zylindrische Außenkontur (148) beschreibt und dadurch einen Durchmesser D148 definiert, und diese zylindrische Außenkontur (148) von den Vertiefungen (143) unterbrochen ist.

Des weiteren zeigen die beiden Abbildungen Fig. 4a und 4b, dass der Gabelkopf (10) in der Seitenansicht u-förmig ist, und in proximaler Richtung (101) zwei Gabelschenkel (111, 112) mit einem innenliegenden Gewinde (113) ausgeformt sind, welche dazu geeignet sind um darin einen Verbindungsstab (40) aufzunehmen, und in dem innenliegenden Gewinde (113) ein Verschlusselement zu führen. Die beiden Gabelschenkel (111, 112) gehen im mittleren Kopfbereich über eine optionale Schräge (114) in einander über. Zentral befindet sich eine Durchgangsöffnung (18). Ebenfalls erkennbar ist, dass der Gabelkopf in seiner bevorzugten Ausführungsform ein oder besser zwei seitlich angeordnete Aussparungen (17) besitzt, die sich von proximal (101->102) kommend entlang, aber im Abstand, zur Zentralachse (103) erstrecken. Dabei sind diese Aussparungen (17) kanalartig oder zumindest teilweise kanalartig ausgebaut, so dass ein längliches Betätigungselement (72) den Gabelkopf (10) zumindest teilweise durchdringen kann, um in Kontakt mit dem Verriegelungsring (20) zu gelangen.

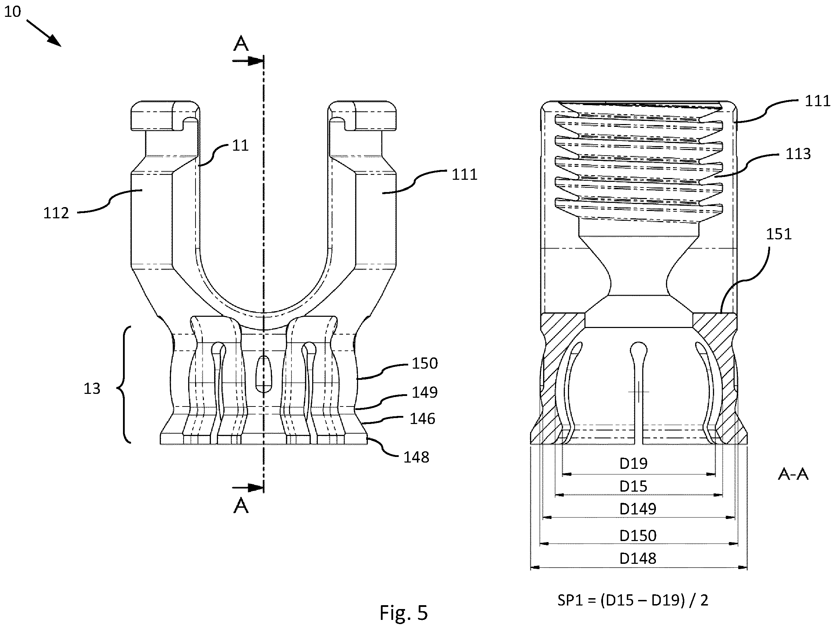

Fig. 5 illustriert den Gabelkopf (10) in einer seitlichen Ansicht und im Schnitt. Hieraus ist zu entnehmen, dass der Gabelkopf (10) am distalen Ende (102) eine Öffnung (19) mit dem Durchmesser D19 für einen Knochenanker (30) besitzt, und einen Kugelsitz (15) mit einem sphärischen Durchmesser D15 ausbildet, wobei der Durchmesser D15 größer als D19 ist, und eine Mindestauslenkung (SP1) der Schenkel (14) erzwungen werden muss, damit der Knochenanker (30) entfernbar oder einsteckbar ist, welche mindestens die Hälfte der Differenz aus D15 und D19 beträgt. Des Weiteren ist der Gabelkopf (10) dadurch charakterisiert, dass der Kugelkopfaufnahmebereich (13) proximal (101) vom zylindrischen Abschnitt (148) aus betrachtet, an einem Verjüngungsbereich (146 bis 149) angrenzt, und dieser un- oder mittelbar an einen konvex gekrümmten Bereich der Außenwand (150) angrenzt und sich dadurch die Durchmesser D148, D149 und D150 ableiten lassen, und der Durchmesser D148 größer als D150 ist, und der Durchmesser D149 ist kleiner als D150 und D148.

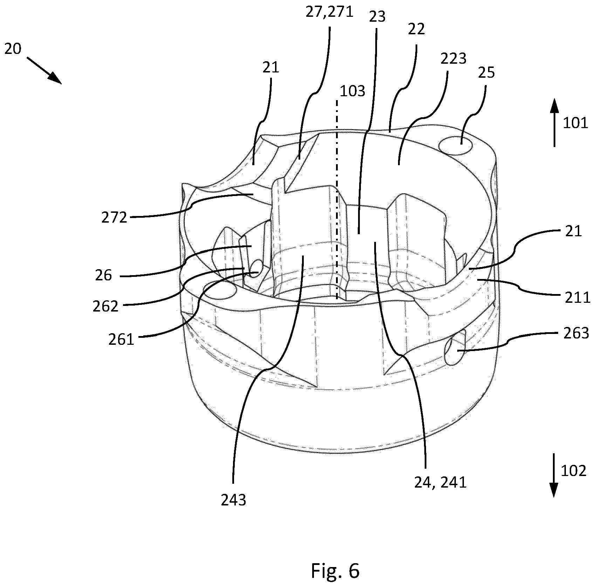

In Fig. 6 ist der Verriegelungsring in einer Schrägansicht illustriert. Zu erkennen ist, dass es zwei Auflage- oder Kontaktflächen (21) gibt, die in Kontakt mit dem Verbindungsstab (40) stehen. Vorzugsweise besitzen diese Auflagefläche an der radial äußeren Kante eine Rundung (211). Dadurch werden höhere Spannungskonzentrationen am Verbindungsstab vermieden. Der Verriegelungsring (20) ist im Wesentlichen konzentrisch aufgebaut, wobei Strukturen oder Ausnehmungen am Randbereich vorgesehen sind, um den Verriegelungsring (20) mit Hilfe von Instrumenten zu greifen und dann zu drehen. Im Inneren besitzt der Verriegelungsring (20) eine zentrale Durchgangsöffnung (23), die, wenn montiert, im Eingriff mit dem Kugelkopfaufnahmebereich (13) des Gabelkopfes (10) steht. Die innere Kontur ist durch Flächenelemente (24, 241) geformt, welche durch Vertiefungen (243) unterbrochen sind. Die Anzahl der Flächenelemente (24, 241) entspricht der Anzahl der am Gabelkopf (10) vorgesehenen Schenkel (14). Das proximale Ende (22) des Verriegelungsrings (20) geht über eine Schräge (223) in die Zentralöffnung über. Außerdem, sind am Verriegelungsring (20) Strukturen (27) vorgesehen, die als Anschläge (271, 272) dienen. Mit diesen Anschlägen wird die Verdrehbarkeit zwischen Gabelkopf und Verriegelungsring eingegrenzt. Des Weiteren zeigt diese Abbildung Fig. 6, dass am oder im Verriegelungsring (20) federelastische Rastelemente (26) vorgesehen sind. Optimalerweise sind sie wie eine Federzunge (261) aufgebaut, welche sich durch Schlitze (262) und einer Kavität (263) ergeben. In der bevorzugten Ausführungsform ist dargestellt, dass der Verriegelungsring (20) am proximalen Ende (22) mindestens eine Kontaktstelle oder Vorsprung (25) vorgesehen ist, damit der Verriegelungsring (20) neben den Stabauflagerstellen (21) auch von der Kontaktstelle oder Vorsprung (25) her betätigt werden kann.

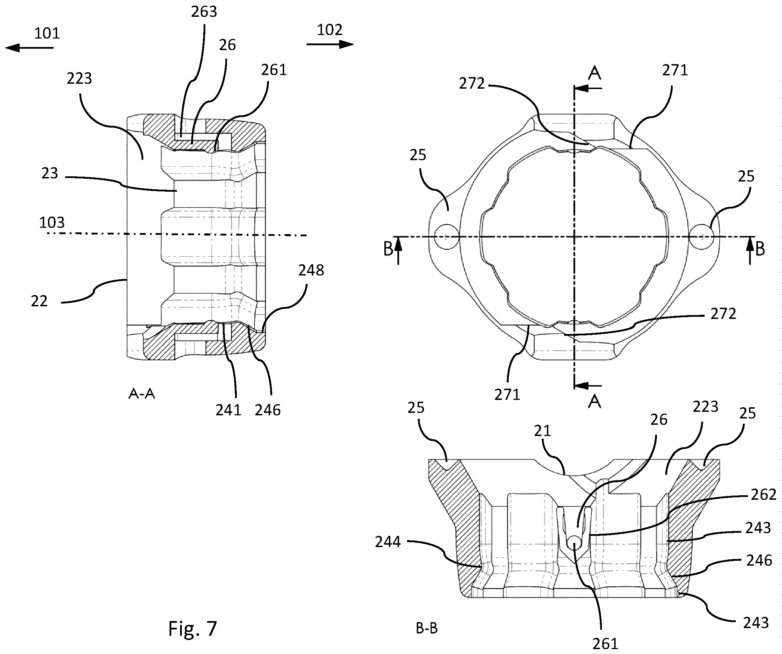

In Fig. 7 dargestellt ist eine Draufsicht auf den Verriegelungsring (20) und zwei erzeugte Schnittansichten. Im Schnitt A-A ist zu erkennen, wie die Rastelemente (26) aufgebaut sind. Ebenfalls gezeigt ist, dass am distalen Ende (102) des Verriegelungsrings (20) die inneren Wandungsabschnitte (241) einen Abschnitt entlang der Zentralachse (103) ausbilden, welcher eine zylindrische Innenkontur (248) mit einem Durchmesser D248 beschreibt, und diese zylindrische Innenkontur (248) von Vertiefungen (243) unterbrochen ist. Insgesamt entspricht die innere Kontur des Verriegelungsrings (20) in etwa einem negativen Abdruck des Kugelkopfaufnahmebereich (13) des Gabelkopfes (10), allerdings mit einem Spalt. In Fig. 7 ist auch abgebildet, dass die Anschläge (271, 272) paarweise und in zwei Richtungen wirksam angeordnet sind.

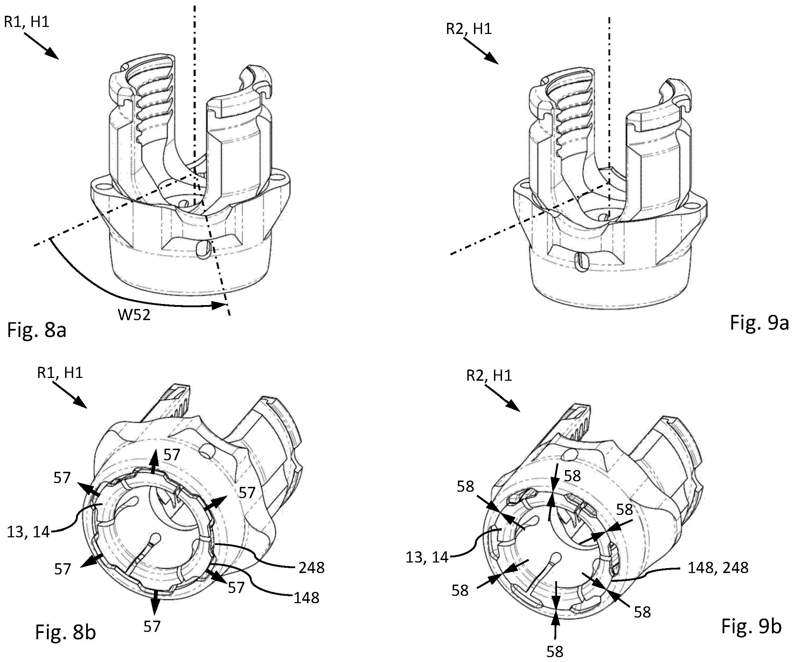

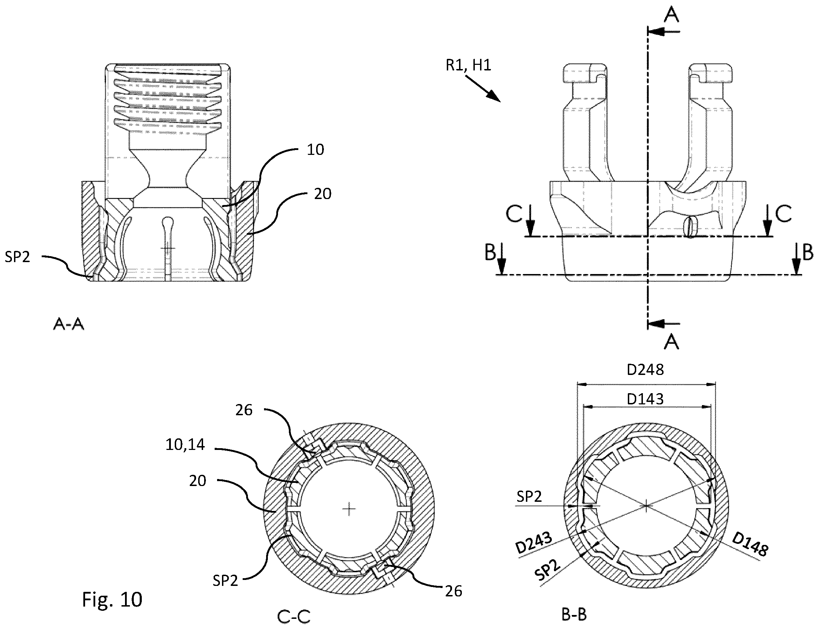

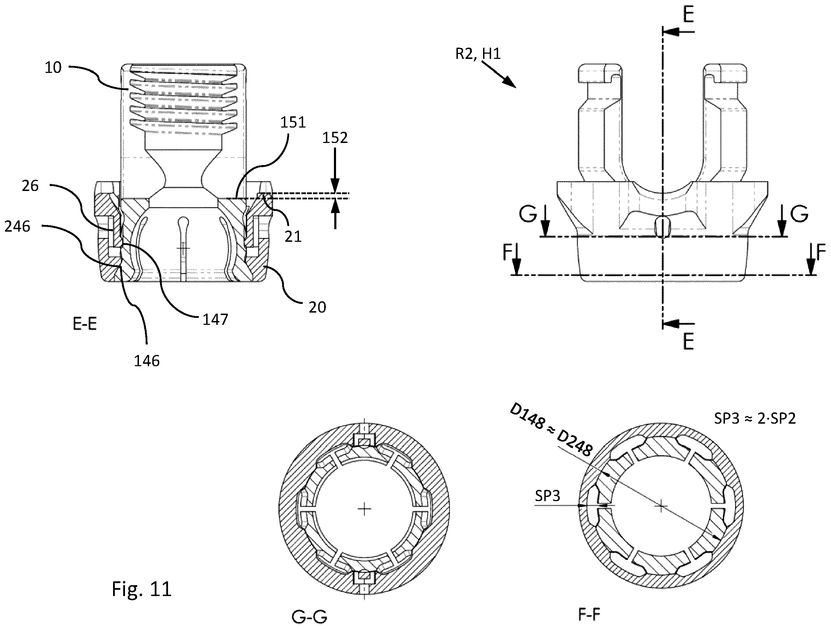

In Fig. 8a, 8b, 9a und 9b ist der funktionale Zusammenhang der Verdrehung des Verriegelungsrings (20) gegenüber dem Gabelkopf (10) und die resultierende Wirksamkeit dargestellt. Der Verriegelungsring (20), ist zumindest teilweise um den Kugelkopfaufnahmebereich (13) herum angebracht, so dass der Verriegelungsring (20) um eine Zentralachse (103) gegenüber dem Gabelkopf (10) drehbar ist. Es kann eine erste Drehstellung (R1) eingestellt werden, bei welcher der Kugelkopfaufnahmebereich (13) mit seinen Schenkeln (14) nach radial außen auslenkbar (57) ist, so dass der Knochenanker (30) entfernbar und/oder einsteckbar ist, und es eine zweite Drehstellung (R2) eingestellt werden kann, bei welcher der Kugelkopfaufnahmebereich (13) mit seinen Schenkeln (14) in seiner Auslenkbarkeit blockiert (58) ist, so dass der Knochenanker (30) ortsfest aber schwenkbar gehalten wird. Beispielsweise ist in Fig. 9b und Fig. 11 zu sehen, dass durch Verdrehen des Verriegelungsrings (20) ein Formschluss (58) zwischen der maximalen äußeren zylindrischen Kontur (148) des Gabelkopfs (10) und der kleinsten zylindrischen Innenkontur (248) des Verriegelungsrings (20) erzeugt wird. In Fig. 8b und Fig. 10 dahingehend ist dargestellt, dass durch Verdrehen in eine andere Richtung der Formschluss wieder geöffnet wird und der Kugelkopfaufnahmebereich (13) verformbar (57) ist. Der Unterschied zwischen der ersten (R1) und zweiten (R2) Drehstellung ist über einen Winkel W52 definierbar, wobei dieser Winkel W52 in etwa dem Quotienten von 180° geteilt durch die Anzahl der Schenkel (14) entspricht (Fig. 8a).

In Fig. 10 auch dargestellt ist, dass die Vertiefungen (143) am Gabelkopf (10) einen Außendurchmesser D143 beschreiben, und die Vertiefungen (243) des Verriegelungsrings (20) einen Innendurchmesser D243 approximieren, und sich eine Durchmesseranordnung (D143, D148, D243 und D248) in der Drehstellung (R1) einstellt, so dass in radialer Richtung (104) ein Spalt (SP2) zwischen den Schenkeln (14) des Gabelkopfs (10) und dem Verriegelungsrings (20) entsteht, so dass die Schenkel (14) nach radial außen auslenkbar (57) sind. Dabei ist der Spalt (SP2) so dimensioniert, dass der Spalt (SP2) gleich oder größer ist als das Maß der Mindestauslenkung (SP1) der Schenkel (14) ist. Erst damit ist sichergestellt, dass der Knochenanker (30) mit seinem Kugelkopf (31) aus dem Kugelsitz (15) entfernbar oder montierbar ist.

In Fig. 11 ist gezeigt, dass der zuvor erwähnte Formschluss durch die Durchmesser D148 und D248 bestimmt wird, die in etwa gleichgroß sind und in der Drehstellung (R2) ein radiales Aufspreizen (58) der Schenkel (14) verhindern. Durch das Verdrehen in die Drehstellung R2, wird der Spalt (SP2) teilweise bzw. segmentweise aufgehoben und resultiert in einen zumindest teilweisen Formschluss, welcher durch die Vertiefungen (143, 243) unterbrochen ist. In diesen Vertiefungsbereichen (143, 243) addieren sich an diesen Stellen die jeweiligen Spalte (SP2) zu einem in etwa doppelt so großen Spalt (SP3) (Fig. 11). Vorzugsweise ist dieser Formschluss nicht nur auf den zylindrischen Anteil des Kugelkopfaufnahmebereichs (13) beschränkt, sondern erstreckt sich auch auf den Konusbereich (146, 246) beider Komponenten. Durch Verdrehen des Verriegelungsrings (20) entsteht ein zusätzlicher Formschluss zwischen der größten äußeren konusartigen Kontur (146) des Gabelkopfs (10) und der kleinsten konusartigen Innenkontur (246) des Verriegelungsrings (20). Durch Verdrehen in eine andere Richtung wird der Formschluss wieder geöffnet (Fig. 10).

In Fig. 11 ist abgebildet, dass der Verriegelungsring (20) in der Drehstellung (R2) eine erste Position (R2, H1) entlang der Zentralachse (103) einnehmen kann, in welcher der Knochenanker (30) ortsfest aber schwenkbar gehalten wird. Wird nun eine axiale Kompressionskraft mit Hilfe des Verbindungstabs (40) auf den Verriegelungsring (20) übertragen wird eine zweite axiale Position (R2, H2) entlang der Zentralachse (103) eingenommen. In dieser Position (R2, H2) wird der Kugelkopfaufnahmebereich (13) vom Verriegelungsring (20) derart zusammengedrückt, so dass der Knochenanker (30) winkelstabil im Kugelsitz (15) gehalten wird. Dabei ist erkennbar, dass die zweite axiale Position (R2, H2) des Verriegelungsrings (20) distal (102) im Bezug zur ersten axialen Position (R2, H1) liegt und die Positionsänderung über eine Wegänderung (152) des Verriegelungsring (20) bestimmbar ist. Die Klemmkraft wird hauptsächlich durch eine paarige Anordnung eines inneren (246) und äußeren Konusbereichs (146) erzeugt. Dabei wird die axial angreifende Kompressionskraft über den Konuswinkel in eine seitlich angreifende und nach innen wirkende Klemmkraft umgeleitet. In Fig. 11 ebenfalls gezeigt ist, dass wenigstens ein federelastisches Rastelement (26) und eine dazu geeignete Rastgegenstelle (147) ausgebildet sind, die miteinander im Eingriff stehen, sobald die Drehstellung (R2) eingestellt ist, um eine Drehbetätigung des Verriegelungsrings (20) aus dieser Drehstellung von R2 nach R1 zu erschweren.

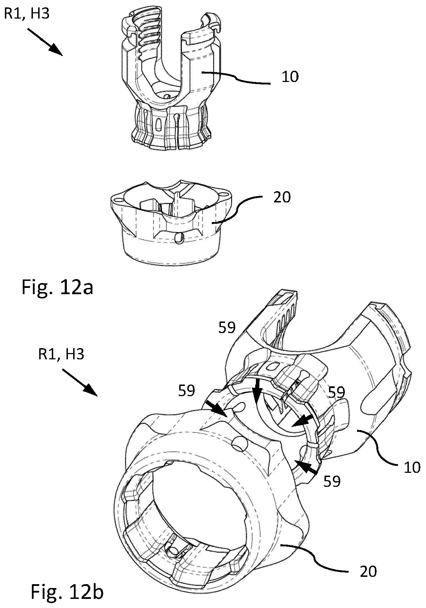

In Fig. 12a und 12b ist die Demontage des Verriegelungsrings (20) vom Gabelkopf (10) dargestellt. Wichtig dabei ist, dass der Verriegelungsring (20) in der Drehstellung (R1) eine erste Position (R1, H1) entlang der Zentralachse (103) einnehmen kann, in welcher der Knochenanker (30) entfernbar und/oder einsteckbar ist, und sobald der Knochenanker (30) aus dem Kugelkopfaufnahmebereich (13) entfernt ist, der Verriegelungsring (20) eine weitere Position (R1, H3) entlang der Zentralachse (103) einnehmen kann, und dadurch der Verriegelungsring (20) entfernbar vom Gabelkopf (10) ist. Voraussetzung ist, dass zum Erreichen der dritten axialen Position (R1, H3) entlang der Zentralachse (103), der Kugelkopfaufnahmebereich (13) mit seinen Schenkeln (14) nach radial innen auslenkt (59), damit der Verriegelungsring (20) entfernbar vom Gabelkopf (10) ist. Dazu muss der Knochenanker (30) aus dem Kugelkopfaufnahmebereich (13) entfernt sein. Dabei ist es so, dass die axiale Position (H2) proximal von der axialen Position (H3) liegt.

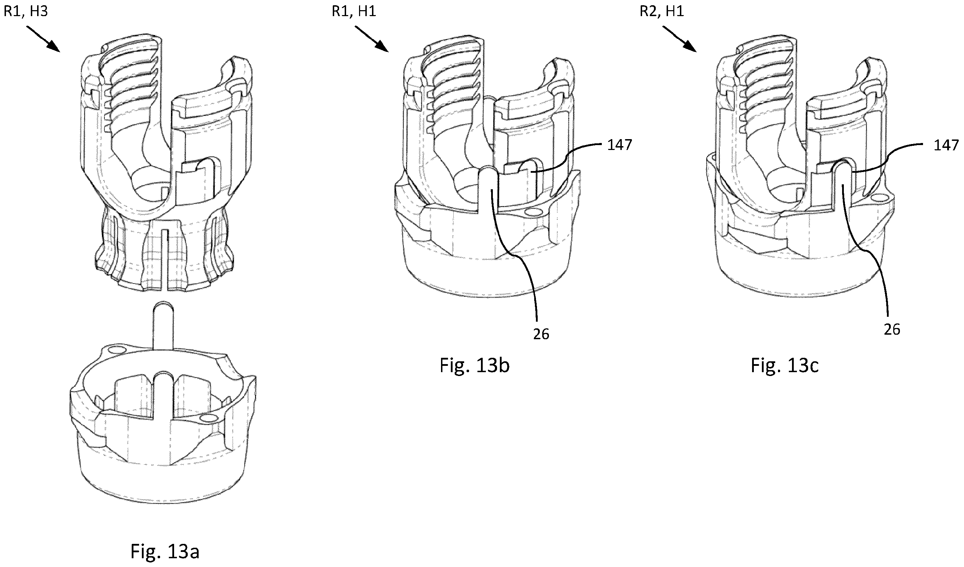

Fig. 13a bis 13c zeigen eine alternative Ausgestaltungsform, bei dem das oder die Rastelemente (26) am proximalen Ende des Verriegelungsrings (20) angeordnet sind. Hierzu ist gegenläufig am Gabelkopf (10) eine Raststelle (147) als vertiefende Aussparung am Gabelkopf vorsehen, in die das Rastelement (26) in der Drehstellung R2 eingreift. Eine gegenläufige Drehung, d.h. R1, ist damit erschwert.

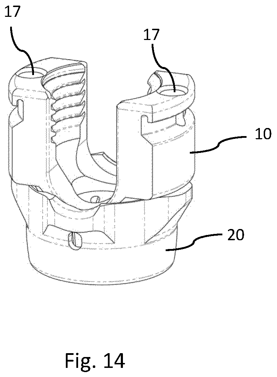

In Fig. 14 ist eine weitere alternative Ausgestaltungsform gezeigt. Diese Ausführungsform besitzt anstatt der teilweisen Aussparungen (17) einen geschlossenen Kanal für die Verwendung eines Instruments (70, 71, 72). Dabei ist es wichtig, dass in der Außenwandung des proximalen Gabelkopfs (10) wenigstens eine Aussparung oder Kanal (17) entlang aber in einem Abstand zur Zentralachse (103) ausgeformt ist, und die Aussparung (17) zur Kontaktstelle oder dem Vorsprung (25) der Verriegelungsrings (20) mündet, so dass ein Betätigungselement (72) von proximal kommend den Gabelkopf (10) vollständig durchdringt, um die Kontaktstelle oder Vorsprung (25) zu erreichen. Somit wird die Führung des Betätigungselements (72) verbessert.

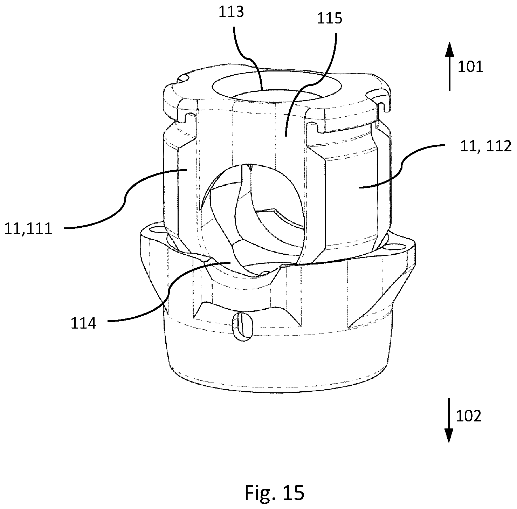

Fig. 15 zeigt eine weitere Ausgestaltungsform bei der die Gabelschenkel (111, 112) am proximalen Bereich (101) miteinander verbunden (115) sind und im Verbund das innenliegende Gewinde (113) enthalten. Dies kann vorteilhaft sein, wenn das Risikos eines Verlusts des Verbindungsstabs minimiert werden soll. Dies kann beispielsweise bei hochbelasteten Knochenankern sehr von Vorteil sein einen nach proximal hin geschlossenen Gabelkopf (10, 115) zu verwenden.

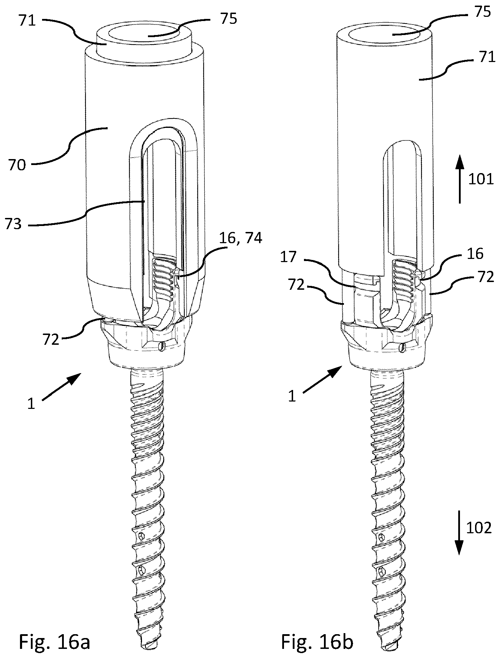

In der bevorzugten Ausgestaltungsform wird durch wenigstens einen zusätzlichen Vorsprung oder Kontaktstelle (25), die mittelbar oder unmittelbar Element der Außenwandung des Verriegelungsrings (20) ist und hierüber eine Kompressionskraft eingeleitet werden kann, eine Möglichkeit bereitgestellt, die Klemmung des Knochenankerkopfes (31) auch ohne eingelegtem Verbindungsstab (40) und/oder Madenschraube (60) zu erwirken (Fig. 16a und 16b). Hierfür ist wie bereits geschildert, an der proximalen Außenwandung (22) des Verriegelungsrings (20) mindestens eine Kontaktstelle oder Vorsprung (25), und am proximalen Gabelkopf (10) zusätzlich mindestens ein Haltemerkmal (16) für die Befestigung eines Instruments (70, 71) vorgesehen. Optional ist am Gabelkopf eine Aussparung (17) vorgesehen, damit die Kontaktstelle (25) für ein Instrument von proximal (101) zugänglich ist. Mit Hilfe eines dafür geeigneten Instruments (70, 71) kann auf die Kontaktstelle (25) eine Druckkraft eingeleitet werden, wobei das Haltemerkmal (16) als Gegenlager für die Reaktions-Zugkraft dient (Fig. 16a und 16b). Zur Einleitung einer Relativbewegung muss das Instrument aus mindestens zwei und gegeneinander verschieblich angeordneten Hülsen (70, 71) bestehen. Hierfür eignet sich eine äußere Hülse (70), die an dem Haltemerkmal (16) des Gabelkopfs (10) durch ein geeignetes Eingriffsmerkmal (74) befestigt werden kann, sowie eine innere Hülse (71), die entsprechende Zapfen oder Vorsprünge besitzt (72), die mit der Kontaktstelle (25) direkt kommuniziert und eine Druckkraft applizieren kann. In Fig. 16b ist die äußere Instrumentenhülse (70) ausgeblendet, damit die Zapfen (72) besser sichtbar sind. Diese Zapfen oder Vorsprünge (72) betätigen den Verriegelungsring (20) von proximal und durchdringen den Gabelkopf (10) zumindest teilweise. Optimalerweise besitzt ein solches Instrument einen seitlichen ovalen Ausschnitt (73) in welcher ein Verbindungsstab (40) eingelegt und geführt werden kann. Bei Einleitung einer Druckkraft zur Kontaktstelle (25) wird eine winkelstabile Klemmung des Knochenankers (30) im Gabelkopf (10, 13) erzeugt, ohne dass ein Verbindungsstab (40) und/oder Madenschraube (60) vorhanden sein muss. Zentral im Instrument (70, 71) befindet sich eine Zentrale Öffnung (75), für den Transport einer weiteren Hülse und/oder Madenschraube (60).

## Patentansprüche

1. Osteosynthesevorrichtung (1), zur Behandlung der Wirbelsäule, umfassend: einen in einer Seitenansicht u-förmigen Gabelkopf (10), der in proximaler Richtung (101) zwei Gabelschenkel (111, 112) mit einem innenliegenden Gewinde (113) aufweist, und darin ein Verbindungsstab (40) aufgenommen werden kann, und in dem innenliegenden Gewinde (113) ein Verschlusselement (60) geführt ist, und der Gabelkopf (10) einen Kugelkopfaufnahmebereich (13) besitzt und lösbar mit einem Knochenanker (30) verbunden ist, und der Knochenanker (30) schwenkbar in einem Kugelsitz (15) des Kugelkopfaufnahmebereichs gelagert (13) ist, wobei der Gabelkopf (10) am Kugelkopfaufnahmebereich (13) nach distal offene Schlitze (12) aufweist und dadurch wenigstens zwei auslenkbare Schenkel (14) ausgebildet sind; und einen Verriegelungsring (20), der zumindest teilweise um den Kugelkopfaufnahmebereich (13) herum angebracht ist, und der Verriegelungsring (20) um eine Zentralachse (103) gegenüber dem Gabelkopf (10) drehbar ist, und eine erste Drehstellung (R1) eingestellt werden kann, bei welcher der Kugelkopfaufnahmebereich (13) mit seinen Schenkeln (14) nach radial außen auslenkbar (57) ist, so dass der Knochenanker (30) entfernbar und/oder einsteckbar ist, **dadurch gekennzeichnet, dass** eine zweite Drehstellung (R2) eingestellt werden kann, bei welcher der Kugelkopfaufnahmebereich (13) mit seinen Schenkeln (14) in seiner Auslenkbarkeit blockiert (58) ist, so dass der Knochenanker (30) ortsfest aber schwenkbar gehalten wird.

2. Osteosynthesevorrichtung (1) nach Anspruch 1, **dadurch gekennzeichnet, dass** der Verriegelungsring (20) in der Drehstellung (R2) eine erste Position (R2, H1) entlang der Zentralachse (103) einnehmen kann, in welcher der Knochenanker (30) ortsfest aber schwenkbar gehalten wird, und eine zweite Position (R2, H2) entlang der Zentralachse (103), wobei in dieser Position (R2, H2) der Kugelkopfaufnahmebereich (13) vom Verriegelungsring (20) derart zusammengedrückt wird, so dass der Knochenanker (30) winkelstabil im Kugelsitz (15) gehalten wird.

3. Osteosynthesevorrichtung (1) nach Anspruch 2, **dadurch gekennzeichnet, dass** die zweite axiale Position (R2, H2) des Verriegelungsrings (20) distal (102) im Bezug zur ersten axialen Position (R2, H1) liegt und die Positionsänderung über eine Wegänderung (152) des Verriegelungsring (20) bestimmbar ist.

4. Osteosynthesevorrichtung (1) nach einem der vorhergehenden Ansprüche, **dadurch gekennzeichnet, dass** der Verriegelungsring (20) in der Drehstellung (52) eine erste Position (R1, H1) entlang der Zentralachse (103) einnehmen kann, in welcher der Knochenanker (30) entfernbar und/oder einsteckbar ist, und der Verriegelungsring (20) eine dritte Position (R1, H3) entlang der Zentralachse (103) einnehmen kann, und dadurch der Verriegelungsring (20) entfernbar vom Gabelkopf (10) ist.

5. Osteosynthesevorrichtung (1) nach Anspruch 4, **dadurch gekennzeichnet, dass** erst bei entferntem Knochenanker (30) aus dem Kugelkopfaufnahmebereich (13), zum Erreichen der dritten Position (R1, H3) entlang der Zentralachse (103), der Kugelkopfaufnahmebereich (13) mit seinen Schenkeln (14) nach radial innen auslenkt (59), damit der Verriegelungsring (20) entfernbar vom Gabelkopf (10) ist.

6. Osteosynthesevorrichtung (1) nach einem der vorhergehenden Ansprüche, **dadurch gekennzeichnet, dass** der Unterschied zwischen der ersten (R1) und zweiten (R2) Drehstellung über einen Winkel W52 definierbar ist, und dieser Winkel W52 in etwa dem Quotienten von 180° geteilt durch die Anzahl der Schenkel (14) entspricht.

7. Osteosynthesevorrichtung (1) nach einem der vorhergehenden Ansprüche, **dadurch gekennzeichnet, dass** wenigstens eine der Drehstellungen (R1, R2) durch einen Anschlag (27, 271, 272) in der Drehung zwischen Verriegelungsring (20) und Gabelkopf (10) limitiert sind.

8. Osteosynthesevorrichtung (1) nach einem der vorhergehenden Ansprüche, **dadurch gekennzeichnet, dass** die Schenkel (14) eine Außenkontur (141) des Kugelkopfaufnahmebereichs (13) beschreiben, und die Schenkel (14) in Umfangsrichtung (105) wenigstens eine Vertiefung (143) besitzen, die sich zumindest abschnittsweise hauptsächlich entlang der Zentralachse (103) erstreckt.

9. Osteosynthesevorrichtung (1) nach einem der vorhergehenden Ansprüche, **dadurch gekennzeichnet, dass** die Vertiefungen (143) durch Schlitze (12) geteilt und/oder unterbrochen sind.

10. Osteosynthesevorrichtung (1) nach einem der vorhergehenden Ansprüche, **dadurch gekennzeichnet, dass** die Schenkel (14) entlang der gedachten Schenkelmittellinie (144) dicker sind als zu den Spalt-angrenzenden Bereichen (12).

11. Osteosynthesevorrichtung (1) nach einem der vorhergehenden Ansprüche, **dadurch gekennzeichnet, dass** am distalen Ende (102) des Gabelkopfes (10) die Schlitze (14) einen Abschnitt entlang der Zentralachse (103) bilden, welcher eine zylindrische Außenkontur (148) beschreibt und dadurch einen Durchmesser D148 definiert, und diese zylindrische Außenkontur (148) von den Vertiefungen (143) unterbrochen ist, und am distalen Ende (102) des Verriegelungsrings (20) die inneren Wandungsabschnitte (241) einen Abschnitt entlang der Zentralachse (103) ausbilden, welcher eine zylindrische Innenkontur (248) mit einem Durchmesser D248 beschreibt, und diese zylindrische Innenkontur (248) von Vertiefungen (243) unterbrochen ist, und die Durchmesser D148 und D248 in etwa gleichgroß sind und in der Drehstellung (R2) ein radiales Aufspreizen der Schenkel (14) verhindern.

12. Osteosynthesevorrichtung (1) nach Anspruch 11, **dadurch gekennzeichnet, dass** die Vertiefungen (143) am Gabelkopf (10) einen Außendurchmesser D143 beschreiben, und die Vertiefungen (243) des Verriegelungsrings (20) einen Innendurchmesser D243 approximieren, und sich eine Durchmesseranordnung (D143, D148, D243 und D248) in der Drehstellung (R1) einstellt, so dass in radialer Richtung (104) ein Spalt (SP2) zwischen den Schenkeln (14) des Gabelkopfs (10) und dem Verriegelungsrings (20) entsteht, so dass die Schenkel (14) nach radial außen auslenkbar (57) sind.

13. Osteosynthesevorrichtung (1) nach einem der vorhergehenden Ansprüche, **dadurch gekennzeichnet, dass** der Kugelkopfaufnahmebereich (13) proximal (101) vom zylindrischen Abschnitt (148) aus betrachtet, an einem Verjüngungsbereich (146 bis 149) angrenzt, und dieser un- oder mittelbar an einen konvex gekrümmten Bereich der Außenwand (150) angrenzt und sich dadurch die Durchmesser D148, D149 und D150 ableiten lassen, und der Durchmesser D148 größer ist als D150, und der Durchmesser D149 kleiner ist als D150 und D148.

14. Osteosynthesevorrichtung (1) nach einem der vorhergehenden Ansprüche, **dadurch gekennzeichnet, dass** am Verriegelungsring (20) nach proximaler Richtung (101) gerichtet mindestens eine Kontaktstelle oder Vorsprung (25) vorgesehen ist, und am proximalen Gabelkopf (10) mindestens ein Haltemerkmal (16) für die Befestigung eines Instruments (70, 74) vorgesehen ist, und bei Einleitung einer Druckkraft zwischen dem Haltemerkmal (16) und der Kontaktstelle (25) eine winkelstabile Klemmung des Knochenankers (30) im Gabelkopf (10, 13) erzeugt wird, ohne dass ein Verbindungsstab (40) und/oder Verschlusselement (60) vorhanden ist.

15. Osteosynthesevorrichtung (1) nach einem der vorhergehenden Ansprüche, **dadurch gekennzeichnet, dass** in der Außenwandung des proximalen Gabelkopfs (10) wenigstens eine Aussparung oder Kanal (17) entlang aber in einem Abstand zur Zentralachse (103) ausgeformt ist, und die Aussparung (17) zur Kontaktstelle oder dem Vorsprung (25) der Verriegelungsrings (20) mündet, so dass ein Betätigungselement (72) von proximal kommend den Gabelkopf (10) wenigstens teilweise durchdringt, um die Kontaktstelle oder Vorsprung (25) zu erreichen.

16. Osteosynthesevorrichtung (1) nach einem der vorhergehenden Ansprüche, **dadurch gekennzeichnet, dass** die auslenkbaren Schenkel (14) von distal kommend über dem Kugelsitz (15) sich vereinen und den mittlere Gabelkopfbereich (114) und die Gabelschenkel (11, 111, 112) ausbilden.

17. Osteosynthesevorrichtung (1) nach einem der vorhergehenden Ansprüche, **dadurch gekennzeichnet, dass** durch Verdrehen des Verriegelungsrings (20) ein Formschluss zwischen der maximalen äußeren zylindrischen Kontur (148) des Gabelkopfs (10) und der kleinsten zylindrischen Innenkontur (248) des Verriegelungsrings (20) erzeugt wird.

## Claims

1. An osteosynthesis device (1) for treating the spine comprising:
a fork head (10) which is U-shaped in a side view, has two fork legs (111, 112) in the proximal direction (101) with an internal thread (113), and in which a connecting rod (40) can be received, and a locking element (60) is guided in the internal thread (113), and the fork head (10) has a spherical head receiving area (13) and is detachably connected to a bone anchor (30), and the bone anchor (30) is pivotably mounted in a spherical seat (15) of the spherical head receiving area (13), wherein the fork head (10) has slots (12) which are open in the distal direction at the spherical head receiving area (13) and at least two deflectable legs (14) are thereby formed; and a locking ring (20) mounted at least partially around the spherical head receiving area (13), and the locking ring (20) is rotatable about a central axis (103) relative to the fork head (10), and a first rotational position (R1) can be set in which the spherical head receiving area (13) can be deflected (57) with its legs (14) radially outwards, so that the bone anchor (30) can be removed and/or inserted,
**characterized in that**
a second rotational position (R2) can be set, in which the spherical head receiving area (13) with its legs (14) is blocked (58) in its deflectability, so that the bone anchor (30) is held fixed but pivotable.

2. The osteosynthesis device (1) according to claim 1, **characterized in that** the locking ring (20) in the rotational position (R2) can take a first position (R2, H1) along the central axis (103), in which the bone anchor (30) is held fixed but pivotable, and a second position (R2, H2) along the central axis (103), in which position (R2, H2) the spherical head receiving area (13) is compressed by the locking ring (20) in such a way that the bone anchor (30) is held in the spherical seat (15) in an angularly stable manner.

3. The osteosynthesis device (1) according to claim 2, **characterized in that** the second axial position (R2, H2) of the locking ring (20) is distal (102) with respect to the first axial position (R2, H1) and the change in position can be determined via a change of displacement (152) of the locking ring (20).

4. The osteosynthesis device (1) according to any preceding claim, **characterized in that** the locking ring (20) in the rotational position (52) can take a first position (R1, H1) along the central axis (103), in which the bone anchor (30) is removable and/or insertable, and the locking ring (20) can take a third position (R1, H3) along the central axis (103), and thereby the locking ring (20) is removable from the fork head (10).

5. The osteosynthesis device (1) according to claim 4, **characterized in that** only when the bone anchor (30) is removed from the spherical head receiving area (13), in order to reach the third position (R1, H3) along the central axis (103), the spherical head receiving area (13) deflects (59) with its legs (14) radially inwards so that the locking ring (20) can be removed from the fork head (10).

6. The osteosynthesis device (1) according to any preceding claim, **characterized in that** the difference between the first (R1) and second (R2) rotational position is determinable by an angle W52, said angle W52 being substantially equal to the quotient of 180° divided by the number of legs (14).

7. The osteosynthesis device (1) according to any preceding claim, **characterized in that** at least one of the rotational positions (R1, R2) is limited in rotation between the locking ring (20) and the fork head (10) by a stop (27, 271, 272).

8. The osteosynthesis device (1) according to any preceding claim, **characterized in that** the legs (14) form an outer contour (141) of the spherical head receiving area (13), and the legs (14) in peripheral direction (105) have at least one recess (143) extending at least in sections mainly along the central axis (103) .

9. The osteosynthesis device (1) according to any preceding claim, **characterized in that** the recesses (143) are divided and/or interrupted by slots (12).

10. The osteosynthesis device (1) according to any preceding claim, **characterized in that** the legs (14) along the imaginary center line (144) are thicker than to the regions (12) adjacent to the slot.

11. The osteosynthesis device (1) according to any preceding claim, **characterized in that** at the distal end (102) of the fork head (10) the slots (14) form a section along the central axis (103) which forms a cylindrical outer contour (148) and thereby determines a diameter D148, and this cylindrical outer contour (148) is interrupted by the recesses (143) and that at the distal end (102) of the locking ring (20) the inner wall sections (241) form a section along the central axis (103) which forms a cylindrical inner contour (248) with a diameter D248, and this cylindrical inner contour (248) is interrupted by recesses (243) that the diameters D148 and D248 are substantially equal and prevent radial spreading of the legs (14) in the rotational position (R2).

12. The osteosynthesis device (1) according to claim 11, **characterized in that** the recesses (143) on the fork head (10) form an outer diameter D143, and the recesses (243) of the locking ring (20) approximate an inner diameter D243, and a diameter configuration (D143, D148, D243 and D248) is established in the rotational position (R1), so that in radial direction (104) a slot (SP2) is provided between the legs (14) of the fork head (10) and the locking ring (20), so that the legs (14) can be deflected (57) radially outwards.

13. The osteosynthesis device (1) according to any preceding claim, **characterized in that** the spherical head receiving area (13), viewed proximally (101) from the cylindrical portion (148), adjoins a tapered area (146 to 149), which is directly or indirectly adjacent to a convexly curved portion of the outer wall (150), thereby allowing the diameters D148, D149 and D150 to be determined, and the diameter D148 is larger than D150, and the diameter D149 is smaller than D150 and D148.

14. The osteosynthesis device (1) according to any preceding claim, **characterized in that** at least one contact position or projection (25) is provided on the locking ring (20) directed in the proximal direction (101), and at least one retaining feature (16) is provided on the proximal fork head (10) for the attachment of an instrument (70, 74), and if a compressive force is applied between the retaining feature (16) and the contact position (25), the bone anchor (30) is clamped in the fork head (10, 13) in an angularly stable manner without a connecting rod (40) and/or locking element (60) being present.

15. The osteosynthesis device (1) according to any preceding claim, **characterized in that** at least one recess or drain (17) is formed in the outer wall of the proximal fork head (10) along but at a distance from the central axis (103), and the recess (17) opens to the contact position or projection (25) of the locking ring (20), so that an actuating element (72) coming from proximal penetrates the fork head (10) at least partially to reach the contact position or projection (25).

16. The osteosynthesis device (1) according to any preceding claim, **characterized in that** the deflectable legs (14) coming from distally join above the spherical seat (15) and form the middle fork head area (114) and the fork legs (11, 111, 112).

17. The osteosynthesis device (1) according to any preceding claim, **characterized in that** a form fit between the maximum outer cylindrical contour (148) of the fork head (10) and the smallest inner cylindrical contour (248) of the locking ring (20) is provided by twisting the locking ring (20).

## Revendications

1. Dispositif d'ostéosynthèse (1), pour le traitement de la colonne vertébrale, comprenant : une fourche (10) en forme de U en vue latérale, qui présente dans la direction proximale (101) deux branches de fourche (111, 112) avec un filetage intérieur (113), et une tige de liaison (40) peut y être reçue, et un élément de fermeture (60) est guidé dans le filetage intérieur (113), et la fourche (10) possède une zone de réception de la tête sphérique (13) et est reliée de manière amovible à un ancrage osseux (30), et l'ancrage osseux (30) est logé (13) de manière pivotante dans un siège sphérique (15) de la zone de réception de la tête sphérique, la fourche (10) présentant des fentes (12) ouvertes distalement sur la zone de réception de la tête sphérique (13) et au moins deux branches (14) pouvant être déviées étant ainsi formées ; et une bague de verrouillage (20) qui est montée au moins partiellement autour de la zone de réception de la tête sphérique (13), et la bague de verrouillage (20) peut tourner autour d'un axe central (103) par rapport à la fourche (10), et une première position de rotation (R1) peut être réglée, dans laquelle la zone de réception de la tête sphérique (13) peut être déviée (57) avec ses branches (14) radialement vers l'extérieur, de sorte que l'ancrage osseux (30) puisse être retiré et/ou inséré, **caractérisé en ce qu'**une deuxième position de rotation (R2) peut être réglée, dans laquelle la zone de réception de la tête sphérique (13) est bloquée (58) avec ses branches (14) dans sa capacité de déviation, de sorte que l'ancrage osseux (30) soit maintenu de manière fixe mais pivotante.

2. Dispositif d'ostéosynthèse (1) selon la revendication 1, **caractérisé en ce que**, dans la position de rotation (R2), la bague de verrouillage (20) peut prendre une première position (R2, H1) le long de l'axe central (103), dans laquelle l'ancrage osseux (30) est maintenu de manière fixe mais pivotante, et une deuxième position (R2, H2) le long de l'axe central (103), la zone de réception de la tête sphérique (13) étant comprimée par la bague de verrouillage (20) dans cette position (R2, H2) de sorte que l'ancrage osseux (30) soit maintenu de manière angulairement stable dans le siège sphérique (15).

3. Dispositif d'ostéosynthèse (1) selon la revendication 2, **caractérisé en ce que** la deuxième position axiale (R2, H2) de la bague de verrouillage (20) est distale (102) par rapport à la première position axiale (R2, H1) et le changement de position peut être déterminé par un changement de course (152) de la bague de verrouillage (20).

4. Dispositif d'ostéosynthèse (1) selon l'une des revendications précédentes, **caractérisé en ce que**, dans la position de rotation (52), la bague de verrouillage (20) peut prendre une première position (R1, H1) le long de l'axe central (103), dans laquelle l'ancrage osseux (30) peut être retiré et/ou inséré, et la bague de verrouillage (20) peut prendre une troisième position (R1, H3) le long de l'axe central (103), et ainsi la bague de verrouillage (20) peut être retirée de la fourche (10).

5. Dispositif d'ostéosynthèse (1) selon la revendication 4, **caractérisé en ce que** ce n'est que lorsque l'ancrage osseux (30) est retiré de la zone de réception de la tête sphérique (13) pour atteindre la troisième position (R1, H3) le long de l'axe central (103), que la zone de réception de la tête sphérique (13) dévie (59) avec ses branches (14) radialement vers l'intérieur, afin que la bague de verrouillage (20) puisse être retirée de la fourche (10).

6. Dispositif d'ostéosynthèse (1) selon l'une des revendications précédentes, **caractérisé en ce que** la différence entre la première (R1) et la deuxième (R2) position de rotation peut être définie par un angle W52, et cet angle W52 correspond approximativement au quotient de 180° divisé par le nombre de branches (14).

7. Dispositif d'ostéosynthèse (1) selon l'une quelconque des revendications précédentes, **caractérisé en ce qu'**au moins l'une des positions de rotation (R1, R2) est limitée en rotation par une butée (27, 271, 272) entre la bague de verrouillage (20) et la fourche (10).

8. Dispositif d'ostéosynthèse (1) selon l'une des revendications précédentes, **caractérisé en ce que** les branches (14) décrivent un contour extérieur (141) de la zone de réception de la tête sphérique (13), et les branches (14) possèdent, dans la direction circonférentielle (105), au moins un renfoncement (143) qui s'étend, au moins par sections, principalement le long de l'axe central (103).

9. Dispositif d'ostéosynthèse (1) selon l'une des revendications précédentes, **caractérisé en ce que** les cavités (143) sont divisées et/ou interrompues par des fentes (12).

10. Dispositif d'ostéosynthèse (1) selon l'une des revendications précédentes, **caractérisé en ce que** les branches (14) sont plus épaisses le long de la ligne médiane imaginaire des branches (144) que vers les zones adjacentes à la fente (12).

11. Dispositif d'ostéosynthèse (1) selon l'une quelconque des revendications précédentes, **caractérisé en ce que**, à l'extrémité distale (102) de la fourche (10), les fentes (14) forment une section le long de l'axe central (103) qui décrit un contour extérieur cylindrique (148) et définit ainsi un diamètre D148, et ce contour extérieur cylindrique (148) est interrompu par les renfoncements (143), et à l'extrémité distale (102) de la bague de verrouillage (20), les parties de paroi intérieure (241) forment une section le long de l'axe central (103) qui décrit un contour intérieur cylindrique (248) avec un diamètre D248, et ce contour intérieur cylindrique (248) est interrompu par des renfoncements (243), et les diamètres D148 et D248 sont approximativement de la même taille et empêchent un écartement radial des branches (14) dans la position de rotation (R2).

12. Dispositif d'ostéosynthèse (1) selon la revendication 11, **caractérisé en ce que** les renfoncements (143) sur la fourche (10) décrivent un diamètre extérieur D143, et les renfoncements (243) de la bague de verrouillage (20) approchent un diamètre intérieur D243, et une disposition des diamètres (D143, D148, D243 et D248) s'établit dans la position de rotation (R1), de sorte qu'une fente (SP2) se forme dans la direction radiale (104) entre les branches (14) de la tête de fourche (10) et la bague de verrouillage (20), de sorte que les branches (14) puissent être déviées (57) radialement vers l'extérieur.

13. Dispositif d'ostéosynthèse (1) selon l'une des revendications précédentes, **caractérisé en ce que** la zone de réception de la tête sphérique (13) est proximale (101) par rapport à la section cylindrique (148) et adjacente à une zone conique (146 à 149), et que celle-ci est adjacente, de manière directe ou indirecte, à une zone de courbure convexe de la paroi extérieure (150), ce qui permet de déduire les diamètres D148, D149 et D150, que le diamètre D148 est supérieur à D150, et que le diamètre D149 est inférieur à D150 et D148.

14. Dispositif d'ostéosynthèse (1) selon l'une des revendications précédentes, **caractérisé en ce qu'**au moins un point de contact ou une saillie (25) est prévu(e) sur la bague de verrouillage (20), orienté(e) dans la direction proximale (101), et **en ce qu'**au moins un élément de retenue (16) est prévu sur la fourche proximale (10) pour la fixation d'un instrument (70, 74), et lors de l'application d'une force de pression entre l'élément de retenue (16) et le point de contact (25), un serrage angulairement stable de l'ancrage osseux (30) est produit dans la tête de fourche (10, 13), sans qu'il y ait une tige de liaison (40) et/ou un élément de fermeture (60).

15. Dispositif d'ostéosynthèse (1) selon l'une des revendications précédentes, **caractérisé en ce qu'**au moins un évidement ou canal (17) est formé dans la paroi extérieure de la fourche proximale (10), le long mais à distance de l'axe central (103), et l'évidement (17) débouche vers le point de contact ou la saillie (25) de la bague de verrouillage (20), de sorte qu'un élément d'actionnement (72) venant de la partie proximale traverse au moins partiellement la fourche (10) pour atteindre le point de contact ou la saillie (25).

16. Dispositif d'ostéosynthèse (1) selon l'une des revendications précédentes, **caractérisé en ce que** les branches (14) pouvant être déformées venant de la partie distale se rejoignent au-dessus du siège sphérique (15) et forment la zone centrale de la fourche (114) et les branches de la fourche (11, 111, 112).

17. Dispositif d'ostéosynthèse (1) selon l'une des revendications précédentes, **caractérisé en ce que** la rotation de la bague de verrouillage (20) permet de créer une liaison par complémentarité de forme entre le contour cylindrique extérieur maximal (148) de la fourche (10) et le contour cylindrique intérieur minimal (248) de la bague de verrouillage (20).
